(19) Europäisches Patentamt
European Patent Office
Office européen des brevets

(11) **EP 3 337 458 B1**

(12) **EUROPÄISCHE PATENTSCHRIFT**

(45) Veröffentlichungstag und Bekanntmachung des Hinweises auf die Patenterteilung:
**16.10.2019 Patentblatt 2019/42**

(21) Anmeldenummer: **16751610.3**

(22) Anmeldetag: **17.08.2016**

(51) Int Cl.:
*A61K 9/14* (2006.01)    *A61K 9/00* (2006.01)
*A61K 9/16* (2006.01)    *A61K 9/48* (2006.01)
*A61K 31/451* (2006.01)    *A61K 31/4515* (2006.01)

(86) Internationale Anmeldenummer:
**PCT/EP2016/069456**

(87) Internationale Veröffentlichungsnummer:
**WO 2017/032650 (02.03.2017 Gazette 2017/09)**

(54) **VERWENDUNG VON WASSERLÖSLICHEN POLYMEREN AUF BASIS VON N-VINYLPYRROLIDON UND ACRYLSÄURE ALS PHARMAZEUTISCHE HILFSTOFFE**

USE OF WATER-SOLUBLE POLYMERS BASED ON N-VINYLPYRROLIDONE AND ACRYLIC ACID AS PHARMACEUTICAL ADJUVANTS

UTILISATION DE POLYMÈRES HYDROSOLUBLES À BASE DE N-VINYLPYRROLIDONE ET D'ACIDE ACRYLIQUE COMME ADJUVANTS PHARMACEUTIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.08.2015 EP 15181992**

(43) Veröffentlichungstag der Anmeldung:
**27.06.2018 Patentblatt 2018/26**

(73) Patentinhaber: **BASF SE**
**67056 Ludwigshafen am Rhein (DE)**

(72) Erfinder:
• **SCHIFFTER, Heiko Alexander**
**51491 Overath (DE)**
• **ANGEL, Maximilian**
**95359 Kasendorf (DE)**
• **KOLTER, Karl**
**67117 Limburgerhof (DE)**
• **GUTH, Felicitas**
**67434 Neustadt (DE)**

(74) Vertreter: **BASF IP Association**
**BASF SE**
**G-FLP-C006**
**67056 Ludwigshafen (DE)**

(56) Entgegenhaltungen:
**EP-A1- 2 140 861    WO-A2-2007/017452**
**US-A- 3 770 683**

• **KASHIF SOHAIL ET AL: "pH-sensitive polyvinylpyrrolidone-acrylic acid hydrogels: Impact of material parameters on swelling and drug release", BJPS BRAZILIAN JOURNAL OF PHARMACEUTICAL SCIENCES, Bd. 50, Nr. 1, 1. März 2014 (2014-03-01), Seiten 173-184, XP55309076, Brazil ISSN: 1984-8250, DOI: 10.1590/S1984-82502011000100018**
• **P Hemalatha ET AL: "Reactivity Ratios of N-Vinylpyrrolidone -Acrylic Acid Copolymer", American Journal of Polymer Science, 1. Januar 2014 (2014-01-01), Seiten 16-23, XP55309061, DOI: 10.5923/j.ajps.20140401.03 Gefunden im Internet: URL:http://article.sapub.org/pdf/10.5923.j .ajps. 20140401.03.pdf [gefunden am 2016-10-10]**

EP 3 337 458 B1

**Beschreibung**

Die vorliegende Erfindung betrifft die Verwendung wasserlöslicher Polymeren auf Basis von N-Vinylpyrrolidon und Acrylsäure als pharmazeutische Hilfsstoffe, insbesondere zur Verbesserung der Löslichkeit von in Wasser schwerlöslichen basischen Wirkstoffen. Die Erfindung betrifft weiterhin feste Lösungen aus basischen Wirkstoffen und wasserlöslich Polymeren, Verfahren zur Herstellung solcher festen Lösungen, polymere Salze, die aus solchen festen Lösungen gewonnen werden können sowie Dosierungsformen, die solche festen Lösungen enthalten.

Zahlreiche Arzneistoffe weisen eine sehr niedrige Wasserlöslichkeit auf und können dadurch nicht aus dem Magen- und Darmtrakt resorbiert werden. Die Folge ist eine sehr niedrige Bioverfügbarkeit.

Zur Verbesserung der Bioverfügbarkeit lassen sich unterschiedliche Ansätze zur Lösung dieses Problems verfolgen.

Bei Arzneistoffen, die eine basische Gruppe besitzen, lassen sich durch Umsetzung mit Säuren entsprechende Salze bilden, die teilweise bessere Löslichkeiten aufweisen. Hierzu werden in der Regel niedermolekulare Säuren oder Laugen verwendet. Die gebräuchlichsten Säuren sind: Salzsäure, Schwefelsäure, Essigsäure, Citronensäure, Weinsäure, Fumarsäure, Maleinsäure, Malonsäure, Bernsteinsäure, Phosphorsäure. Häufig besteht kaum ein Unterschied zwischen der Löslichkeit der Arzneistoffsäure oder -base und der eines Salzes mit den genannten Verbindungen. Die Ursache dieser schlechten Löslichkeit liegt in der Regel darin, dass das Salz ein sehr stabiles Kristallgitter ausbildet, das sich energetisch in einem günstigen Zustand befindet, wodurch die Tendenz in Lösung zu gehen niedrig ist. Falls zusätzlich der Energiegewinn durch die Hydratation niedrig ist, wird die Löslichkeit weiter herabgesetzt.

Eine weitere Möglichkeit, das Problem der schlechten Löslichkeit zu überwinden, ist die Bildung von Salzen der schwerlöslichen Arzneistoffe mit polymeren Säuren oder Basen. Solche Salze wurden bisher im Prinzip schon hergestellt, jedoch wurden häufig Polymere eingesetzt, die nicht über einen großen pH-Bereich löslich waren - insbesondere nicht im physiologisch bedeutsamen Bereich von pH 1-8 - oder die in Lösung als Säure, Base oder Salz eine hohe Viskosität aufwiesen. Dadurch wird die Freisetzung verhindert oder zumindest stark verlangsamt.

Weisen die Polymere jedoch eine hohe Viskosität in wässriger Lösung auf, wird die Wirkstofffreisetzung aus einer festen Darreichungsform wie z.B. einer Tablette ebenfalls verzögert. Bei der Auflösung des Salzes entsteht an der Oberfläche der Tablette und in den Hohlräumen ein Gel bzw. eine hochviskose Lösung, die das weitere Vordringen des Wassers in den Tablettenkern behindert und den Zerfall verlangsamt. Durch diesen Effekt wie auch durch den verringerten Diffusionskoeffizienten der Arzneistoffmoleküle durch Areale mit hoher Viskosität wird die Freisetzung des Arzneistoffes verzögert. Insofern sind gelbildende Polymere zur Herstellung von schnellfreisetzenden Formen ungeeignet, bei denen ein schwerlöslicher Arzneistoff schnell aufgelöst werden soll und der gesamten Magen- und Dünndarmoberfläche zur Resorption dargeboten werden soll.

In EP 0211268 werden Minoxidilsalze mit polymeren Polyanionen beschrieben, die eine Freisetzungsverzögerung aufweisen und zur dermalen Applikation verwendet werden. Minoxidil ist ein Arzneistoff der 4 zur Salzbildung befähigte Gruppen enthält und die entsprechenden polymeren Salze waren schlechter löslich als das Hydrochlorid. Durch die zahlreichen zur Salzbildung befähigten Gruppen ist die Dissoziation des Salzes stark vermindert und die Löslichkeit gegenüber dem Hydrochlorid nicht verbessert. Orale Applikationen sind nicht beschrieben.

In US 4997643 wird ein biokompatibles, filmbildendes Delivery System für die topische Applikation beschrieben, das ein polymeres Salz mit einer carboxylgruppentragenden Komponente beinhaltet. Als Arzneistoff wird ebenfalls Minoxidil eingesetzt, der die o.g. Besonderheiten aufweist. Orale Anwendungen sind nicht beschrieben.

In US 4248855 werden flüssige Zubereitungen beansprucht, die Salze aus basischen Arzneistoffen und wasserunlöslichen Polymeren enthalten, und die einen Retardeffekt besitzen. Durch die Verwendung von wasserunlöslichen Polymeren zeigen die Zubereitungen keine schnelle Freisetzung und keine hohe Löslichkeit über einen großen pH-Bereich.

Aus der US 5736127 ist bekannt, dass aus basischen Arzneistoffen und Polymeren, die durch partielle Hydrolyse von gelbildenden Polyacrylnitrilen erhalten werden, Salze gebildet werden können. Aufgrund der hohen Molekulargewichte sind die Polymeren gelbildend, wodurch die Freisetzung der Wirkstoffe verzögert wird. Eine Eignung für schnell freisetzende Tabletten ist nicht gegeben.

In der US 4205060 werden Mikrokapseln mit verzögerter Freisetzung beschrieben, die im Kern ein Salz eines basischen Arzneistoffes mit einem carboxylgruppenhaltigem Polymer enthalten und der mit einem wasserunlöslichen Polymer umhüllt ist. Durch das carboxylgruppenhaltige Polymer wird die Freisetzung der verwendeten löslichen Arzneistoffe vermindert.

Salze des Ranitidins mit Polycarbonsäuren sind in EP 0721785 beschrieben. Die Polycarbonsäuren binden das Ranitidin und sollen dessen Bitterkeit herabsetzen. Allerdings sind niedermolekulare Salze des Ranitidin gut löslich, so dass die Polycarbonsäuren lediglich die Beweglichkeit und Diffusion des Ranitidins einschränken, so dass es nicht so schnell an die Bitterrezeptoren gelangt.

Um solche polymeren Salze bilden zu können, muss der Wirkstoff jedoch eine gewisse Restlöslichkeit in einem wässrigen oder wässrig-organischen System aufweisen, was oft nicht gegeben ist.

In der US 4,853,439 werden wasserlösliche Komplexe von Homo- oder Copolymeren des N-Vinylpyrrolidons mit wasserunlöslichen Wirkstoffen beschrieben. Unter den Copolymeren sind ganz allgemein solche beschrieben, die säuregruppenhaltige Monomere wie Acrylsäure enthalten können. Konkret beschrieben ist jedoch nur ein Copolymer mit Maleinsäureanhydrid, dass aufgrund physikalischer Eigenschaften wie Farbe, Reinheit und Viskosität für den pharmazeutischen Anwender nicht akzeptabel ist.

H. Uelzmann, Journal of Polymer Science, Vol. XXXIII, S. 377-379 (1958) beschreibt ohne Angaben zu einer möglichen Verwendung relativ höhermolekulare Copolymere aus Acrylsäure und N-Vinylpyrrolidon mit unterschiedlichen molaren Anteilen, wobei solche Copolymere mit molaren Verhältnissen von 1:1 oder 1:2 AA:VP zwar in konzentrierter Form (50 gew.-ig) in Wasser löslich sind. Bei Verdünnung mit Wasser auf beispielsweise 5 gew.-ige Mischungen, fällt das Copolymer jedoch aus. Die Copolymeren sind auch in verdünnter Salzsäure nicht löslich. Ein solches Löslichkeitsverhalten ist für die Anwendung zur Löslichkeitsverbesserung von pharmazeutischen Wirkstoffen nicht akzeptabel.

Eine andere Möglichkeit, die Löslichkeit schwerlöslicher Arzneistoffe zu verbessern, besteht in der Herstellung sogenannter "fester Lösungen" der Arzneistoffe in einer Polymermatrix. In solchen festen Lösungen liegt der Arzneistoff molekulardispers in der Polymermatrix verteilt vor. Siehe zum Beispiel: Janssens S. and Van den Mooter G.,: Review: Physical chemistry of solid dispersions, Journal of Pharmacy and Pharmacology 61, 1571-1586 (2009); Vasconcelos et al., Solid dispersions as strategy to improve oral bioavailability of poor water soluble drugs, Drug Discovery Today, Volume 12, Numbers 23/24, 1068-1075, (2006).

Solche festen Lösungen können aus organischen Lösungen beider Komponenten durch Verdampfen des Lösungsmittels oder durch Schmelzextrusion erhalten werden. Jedoch sind manche Wirkstoffe so schwer löslich, dass sie oft auch in solchen Lösungsmittels, die für die technische Anwendung akzeptabel sind, nicht in ausreichendem Maße gelöst werden können. Dann bleibt als alternative Herstellungsweise nur die Schmelzextrusion, bei der allerdings gewisse Anforderungen an die Temperatur- und Scherstabilität der eingesetzten Komponenten gestellt werden müssen. WO 2007/017452 offenbart die Verwendung von Copolymeren, enthaltend a) 60 bis 99 Gew.-% N-Vinylcaprolactam; b) 1 bis 40 Gew.-% mindestens eines Monomeren b) ausgewählt aus der Gruppe der: b1) C8-C30-Alkylester von monoethylensich ungesättigten C3-C8-Carbonsäuren; b2) N-Alkyl- oder N,N-Dialkylsubstituierten Amide der Acrylsäure oder der Methacrylsäure mit C8 bis C30-Alkylresten; b3) der Vinylester von aliphatischen unverzweigten C8-C30-Carbonsäuren; b4) der C8-C30-Alkyl-Vinylether, als Solubilisatoren für in Wasser schwerlösliche Substanzen.

Aus der WO 2009/074609 sind polymere Wirkstoffsalze bekannt, die einen hohen Anteil an säuregruppenhaltigen Monomeren aufweisen. Konkret beschrieben sind polymere Gegenionen, die mindestens 80 Gew.-% an carboxylgruppenhaltigen Monomeren wie der Acrylsäure enthalten. Es hat sich jedoch gezeigt, dass diese Polymere trotz ihrer guten Löslichkeit in der Anwendung Nachteile aufweisen. So neigen sie bei Temperaturbelastung zur Decarboxylierung. Insbesondere bei der Verarbeitung in der Schmelze im Extruder kommt es zu einer Zersetzung der Polymere durch Decarboxylierung. Auch sonst weisen sie hinsichtlich der Lagerstabilität Nachteile auf. Durch den hohen Carboxylgruppengehalt besteht ein größeres Risiko der Wechselwirkung der überschüssigen sauren Gruppen und damit der Schädigung der Wirkstoffe an sensiblen Bindungsstellen.

Ein weiteres Problem besteht darin, dass für wasserempfindliche Wirkstoffe eine Überführung in Hydrochloride sich nicht empfiehlt.

Mangelnde Stabilität des Wirkstoffs, der Formulierungshilfsstoffe oder der Wirkstoffformulierung gegen Zersetzung oder Verfärbung der Formulierungen sind aus Sicht eines pharmazeutischen Unternehmers nicht akzeptabel, ebenso wenig wie im Nahrungsergänzungsmittel- oder Tierfutterbereich oder bei Kosmetika.

Aufgabe der vorliegenden Erfindung war es, Wirkstoffformulierungen zu finden, die die Nachteile des Standes der Technik zu vermeiden helfen und bei guter Stabilität eine im Vergleich zum entsprechenden Hydrochloridsalz schnellere Freisetzung des Wirkstoffs ermöglichen. Weiterhin sollten die gesuchten Formulierungen auch pH-unabhängig löslich sein. Ausserdem sollten die Formulierungen keine konzentrationsabhängigen Mischungslücken in verdünnten Lösungen aufweisen.

Demgemäß wurde die Verwendung eines wasserlöslichen Polymers, welches im pH-Bereich von 1 bis 13 eine Wasserlöslichkeit größer von 5 % (m/m) aufweist und durch radikalisch initiierte Polymerisation einer Monomerenmischung aus i) 70 bis 90 Gew.-% N-Vinylpyrrolidon und ii) 10 bis 30 Gew.-% Acrylsäure, wobei die Summe von i) und ii) 100 Gew.-% entspricht, erhalten wird, zur Formulierung von in Wasser schwerlöslichen basischen Wirkstoffen gefunden, wobei die Wirkstoffe in nicht geladener Form oder als Hydrochlorid eine Löslichkeit von kleiner 0.1 % (m/m) in Wasser, künstlichem Darmsaft oder Magensaft aufweisen.

Gemäß einer bevorzugten Ausführungsform betrifft die Erfindung die Verwendung eines wasserlöslichen Polymers, welches im pH-Bereich von 1 bis 13 eine Wasserlöslichkeit größer von 5 % (m/m) aufweist und durch radikalisch initiierte Polymerisation einer Monomerenmischung aus i) 70 bis 90 Gew.-% N-Vinylpyrrolidon und ii) 10 bis 30 Gew.-% Acrylsäure, wobei die Summe von i) und ii) 100 Gew.-% entspricht, erhalten wird, zur Formulierung von in Wasser schwerlöslichen basischen Wirkstoffen, welche in nicht geladener Form oder als Hydrochlorid eine Löslichkeit von kleiner 0.1 % (m/m) in Wasser, künstlichem Darmsaft oder Magensaft aufweisen, zum Zwecke der Solubilisierung der basischen Wirkstoffe in wässrigem Milieu. Solubilisierung bedeutet erfindungsgemäß, dass die Wasserlöslichkeit der Wirkstoffe in der Formulierung größer ist als die Löslichkeit der entsprechenden Hydrochlorid-Salze.

Gemäß einer weiteren Ausführungsform werden die erfindungsgemäßen wasserlöslichen Polymere als Bindemittel bei der Herstellung fester Dosierungsformen von in Wasser schwerlöslichen basischen Wirkstoffen, welche in nicht geladener Form oder als Hydrochlorid eine Löslichkeit von kleiner 0.1 % (m/m) in Wasser, künstlichem Darmsaft oder Magensaft aufweisen, verwendet.

Gemäß einer besonders bevorzugten Ausführungsform betrifft die Erfindung feste Lösungen von in Wasser schwerlöslichen basischen Wirkstoffen, welche in nicht geladener Form oder als Hydrochlorid eine Löslichkeit von kleiner 0.1 % (m/m) in Wasser, künstlichem Darmsaft oder Magensaft aufweist, und einem wasserlöslichen Polymer, welches im pH-Bereich von 1 bis 13 eine Wasserlöslichkeit von größer 10 % (m/m) aufweist und durch radikalisch initiierte Polymerisation einer Monomerenmischung aus i) 70 bis 90 Gew.-% N-Vinylpyrrolidon und ii) 10 bis 30 Gew.-% Acrylsäure, wobei die Summe von i) und ii) 100 Gew.-% entspricht, erhalten wird. Die erfindungsgemäßen Formulierungen sind wasserlöslich und können sich in Wasser bei Standardbedingungen vollständig unter Bildung von optisch klaren Lösungen auflösen. Die festen Lösungen weisen in Wasser bei Standardbedingungen eine Löslichkeit auf, die größer ist als die der entsprechenden Hydrochloride. Standardbedingungen bedeutet bei 20 +/- 5 °C und Standardluftdruck von 1013,25 hPa.

Die erfindungsgemäßen Formulierungen führen nicht nur zu einer erhöhten Löslichkeit, sondern auch zu einer schnelleren Wirkstofffreisetzung in üblichen Freisetzungsmedien wie Wasser, künstlichem Magen- oder Darmsaft. Dadurch wird in der Regel auch eine höhere Bioverfügbarkeit erzielt.

Weiterhin wurden Verfahren zur Herstellung der festen Lösungen sowie deren Verwendung in pharmazeutischen Dosierungsformen gefunden.

"Formulierung" bedeutet eine physikalische Mischung, die die Verabreichung als Arzneimittel ermöglicht und die Bioverfügbarkeit verbessert. Im Wesentlichen liegen keine starken intermolekularen Wechselwirkungen zwischen den wasserlöslichen Polymeren und den basischen Wirkstoffen vor. "Keine starken intermolekularen Wechselwirkungen" bedeutet, dass zwischen den Molekülen als Wechselwirkungen nur schwache Wechselwirkungen wie van-der Waals-Wechselwirkungen, Wasserstoffbrückenbindungen oder Komplexbindungen auftreten.

Geeignete Wirkstoffe im Sinne der Erfindung sind basische, in Wasser schwerlösliche Wirkstoffe.

"Basisch" bedeutet, dass der Wirkstoff in der Lage ist Kationen zu bilden und" in Wasser schwerlöslich" bedeutet, dass die Wirkstoffe im Sinne der Erfindung solche sind, die in nicht geladener Form oder als Hydrochlorid Löslichkeiten von kleiner 0,1% (m/m) in Wasser, künstlichem Darmsaft oder Magensaft aufweisen (bei 20 +/- 5 °C und Standardluftdruck von 1013,25 hPa) .

Prinzipiell sind alle in Wasser schwerlöslichen pharmazeutischen Wirkstoffe, Nutraceuticals, Lebensmittel- oder Futtermittelzusatzstoffe geeignet, die eine ausreichende Basizität aufweisen. Bevorzugte basische Wirkstoffe weisen mindestens eine und höchstens zwei zur Salzbildung befähigten Gruppen auf.

Insbesondere handelt es sich bei den basischen Wirkstoffen um pharmazeutische Wirkstoffe.

Erfindungsgemäß lassen sich auch Arzneistoffe in Lösung bringen, bei denen weder die neutrale Form noch die entsprechenden niedermolekularen Salze wasserlöslich sind. Bei diesen Arzneistoffen ist dann die Auflösung im Magen- und Darmtrakt sehr langsam und somit limitierend für die Resorption, wodurch häufig eine niedrige Bioverfügbarkeit resultiert (gemäß dem Biopharmaceutical Classification System: Wirkstoffe der Klasse II (Amidon et al.,Pharm. Res. 12, 413-420)).

Die pharmazeutischen Wirkstoffe können dabei aus jedem Indikationsbereich kommen.

Als Beispiele seien hier Antihypertensiva, Vitamine, Cytostatika - insbesondere Taxol, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantien, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Abmagerungsmittel durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten oder antivirale Wirkstoffe oder Wirkstoffe zur Behandlung von HIV-Infektionen und des AID-Syndroms genannt.

Die erfindungsgemäß verwendeten wasserlöslichen Polymere sind alle pH-unabhängig im gesamten pH-Bereich von pH 1 bis pH 13 wasserlöslich. Wasserlöslich bedeutet, dass mindestens 5 % (m/m) Polymer in Wasser klar löslich sind. In den klaren Lösungen ist optisch keine Trübung zu erkennen.

Insbesondere weisen die wasserlöslichen Polymere im pH-Bereich von 3 bis 11 eine Wasserlöslichkeit größer 10 % (m/m) auf.

Alle Angaben zur Wasserlöslichkeit beziehen sich bei allen erfindungsgemäß verwendeten wasserlöslich Polymeren auf Standardbedingungen von 20 +/- 5 °C und Standardluftdruck von 1013,25 hPa. Die Angabe (m/m) bedeutet Massenanteile.

Die erfindungsgemäßen wasserlöslichen Polymere weisen in Wasser und in 0.08 m Salzsäure als Lösemittel im Konzentrationsbereich von 5 Gew.-% bis 50 Gew.-%, bezogen auf Polymer in Lösemittel, keine Mischungslücke auf. "Keine Mischungslücke" bedeutet, dass die Polymere im Lösemittel klar löslich sind, dass also optisch keine Trübung zu erkennen ist.

Die erfindungsgemäß verwendeten wasserlöslichen Polymere bilden in Wasser optisch klare Lösungen aus, die auch lagerstabil sind. Auch bei Lagerung in dem oben erwähnten Konzentrationsbereich bei 40 °C und Standarddruck nach sechs Monaten weisen sie keinen Bodensatz auf. "Kein Bodensatz" bedeutet, dass weniger als 1 Gew.-% des bei der Herstellung der Lösung eingesetzten Polymers aus der Lösung ausfallen. Bevorzugt sind wasserlösliche Polymere, die bei Lagerung unter den genannten Bedingungen in einem wässrigen Lösungsmedium zu weniger als 0.1 Gew.- %, bezogen auf die Menge des eingesetzten wasserlöslich Polymers, ausfallen.

Alle genannten wasserlöslichen Polymere sind in Lösungsmitteln, insbesondere in Wasser, nicht gelbildend. Nicht gelbildend bedeutet, dass sie keine dreidimensionalen Netzwerke ausbilden und daher keine Poren enthalten, in denen sich Lösungsmittelmoleküle einlagern könnten. Die wasserlöslichen Polymere weisen K-Werte nach Fikentscher, gemessen in einer 5 gew-%igen wässrigen Lösung von kleiner 30, besonders bevorzugt kleiner 20 auf. Dieser K-Wert ist eine Kennzahl für die Viskosität der Lösung, die wiederum bei diesen Polymeren ein Maß für das Molekulargewicht darstellt.

Die Glasübergangstemperaturen berechnet nach der FOX-Gleichung liegen im Bereich von 140 bis 160 °C:

$$\frac{1}{T_G} = \sum_i^n x_i \frac{1}{T_{G,i}}$$

$x_i$= Massenanteil des Comonomers im Polymer

$T_{G,i}$= Glasübergangstemperatur des Homopolymers des entsprechenden Comonomers

$T_G$= Glasübergangstemperatur des Copolymers

Die Glasübergangstemperaturen können auch mittels Differential Scanning Calorimetrie bei einer Heizrate von 20 K/min gemessen werden und liegen im Bereich von 130 bis 170 °C.

Wie bereits erwähnt kommen als wasserlösliche Polymere für die wasserlöslichen Formulierungen von in Wasser schwerlöslichen Wirkstoffen, bestehend aus einem basischen Wirkstoff, welcher in nicht geladener Form oder als Hydrochlorid eine Löslichkeit von kleiner 0.1 % (m/m) in Wasser, künstlichem Darmsaft oder Magensaft aufweist, solche Polymere in Betracht, welche im gesamten pH-Bereich von 1bis 13 eine Wasserlöslichkeit größer von 10 % (m/m) aufweisen und durch radikalisch initiierte Polymerisation einer Monomerenmischung aus i) 70 bis 90 Gew.-% N-Vinylpyrrolidon und ii) 10 bis 30 Gew.-% Acrylsäure, wobei die Summe von i) und ii) 100 Gew.-% entspricht, erhalten werden.

Bevorzugt werden wasserlösliche Polymere, welche im gesamten pH-Bereich von 1 bis 13 eine Wasserlöslichkeit größer von 10 % (m/m) aufweisen und durch radikalisch initiierte Polymerisation einer Monomerenmischung aus i) 75 bis 85 Gew.-% N-Vinylpyrrolidon und ii) 15 bis 25 Gew.-% Acrylsäure, wobei die Summe von i) und ii) 100 Gew.-% entspricht, erhalten werden.

Besonders bevorzugt wird ein wasserlösliches Polymer verwendet, welche im gesamten pH-Bereich von 1 bis 13 eine Wasserlöslichkeit größer von 10 % (m/m) aufweist und durch radikalisch initiierte Polymerisation einer Monomerenmischung aus i) 80 Gew.-% N-Vinylpyrrolidon und ii) 20Gew.-% Acrylsäure, wobei die Summe von i) und ii) 100 Gew.-% entspricht erhalten wird.

Die Herstellung der erfindungsgemäßen Polymere kann auf an sich übliche Weise durch radikalische Polymerisation erfolgen. Vorzugsweise erfolgt die Polymerisation als Lösungspolymerisation in organischen Lösungsmitteln, vorzugsweise in Alkoholen, insbesondere in Isopropanol. Solche Verfahren sind dem Fachmann an sich bekannt. Geeignete Initiatoren sind beispielsweise organische Peroxide wie tertiär-Butylperpivalat oder alkohollösliche Azostarter.

Die Polymerisation kann bei Temperaturen von 50 bis 130 °C und Drücken von 0,1 bis 1,5 MPa erfolgen.

Es kann sich auch empfehlen, die Polymerisation in Gegenwart von Reglern, beispielsweise von Natriumhypophosphit, durchzuführen.

Die Polymerisation kann kontinuierlich oder als Batch-Verfahren erfolgen, bevorzugt werden die Polymere über Zulaufverfahren erhalten.

Gemäß einer Ausführungsform der Erfindung wird zunächst das Natriumsalz des Copolymers durch radikalische Copolymerisation von N-Vinylpyrrolidon und Natriumacrylat hergestellt, welches dann durch Ionenaustausch in das freie saure Copolymer überführt werden kann.

Die Überführung der Polymerlösungen in die feste Form kann ebenfalls nach üblichen Trocknungsverfahren wie zum Beispiel durch Sprühtrocknung, Gefriertrocknung oder Walzentrocknung erfolgen.

Gemäß einer besonders bevorzugten Ausführungsform weisen die polymere K-Werte nach Fikentscher, gemessen in einer 5 gew-%igen wässrigen Lösung, im Bereich von 10 bis kleiner 20 auf.

Bei der Herstellung der erfindungsgemäßen Polymeren ist vorzugsweise darauf zu achten, dass diese keine niedermolekulare Anionen wie z. B. Chlorid, Sulfat, etc. aufweisen, die zu schwerlöslichen Salzen mit Wirkstoffen führen können.

Bevorzugt werden die so erhaltenen wasserlöslich Polymere zur Herstellung von festen Lösungen mit basischen in Wasser schwerlöslichen Wirkstoffen verwendet.

Gemäß einer Ausführungsform der Erfindung kann die Herstellung der erfindungsgemäßen festen Lösungen mit Hilfe des Lösungsmittelverfahrens erfolgen.

Der Wirkstoff und das Polymer werden in organischen Lösungsmitteln oder Lösungsmittelgemischen gelöst und die Lösung wird anschließend getrocknet. Das Lösen kann auch bei erhöhten Temperaturen (30 - 200°C) und unter Druck erfolgen.

Geeignete organische Lösungsmittel sind Dimethylformamid, Methanol oder Mischungen davon sowie Mischungen mit Isopropanol, Tetrahydrofuran, Aceton oder Ethylacetat.

Prinzipiell sind alle Trocknungsarten möglich, wie z.B. Sprühtrocknung, Wirbelschichttrocknung, Schaufeltrocknung, Gefriertrocknung, Vakuumtrocknung, Bandtrocknung, Walzentrocknung, Schleppgastrocknung, Eindampfen etc.

Gemäß einer bevorzugten Ausführungsform der Erfindung erfolgt die Herstellung der festen Lösungen durch Schmelzeverfahren. Der Wirkstoff wird mit dem Polymer gemischt. Durch Erhitzen auf Temperaturen von 50 - 250°C erfolgt die Herstellung der festen Lösung. Hierbei sind Temperaturen oberhalb der Glasübergangstemperatur des Polymers oder des Schmelzpunktes des Wirkstoffes vorteilhaft. Durch Zugabe eines weichmachenden Hilfsstoffes wie z. B. Wasser, organisches Lösungsmittel, übliche organische Weichmacher lässt sich die Verarbeitungstemperatur entsprechend absenken. Besonders vorteilhaft sind hier Hilfsstoffe, die nachher recht einfach wieder abgedampft werden können, d.h. einen Siedepunkt unter 180°C bevorzugt unter 150°C aufweisen.

Gemäß einer bevorzugten Ausführungsform wird diese Art der Herstellung in einem Schneckenextruder vorgenommen. Welche Verfahrensparameter dabei im Einzelnen angepasst werden müssen, kann der Fachmann durch einfache Versuche im Rahmen seines üblichen Fachwissens ermitteln.

Gemäß einer bevorzugten Ausführungsform werden während des Aufschmelzens Weichmacher zugesetzt. Bevorzugte Weichmacher sind Zitronensäureester wie beispielsweise Triethylzitrat oder Acetyltributylzitrat), Glykolderivate wie beispielsweise. Polyethylenglykol, Propylenglykol oder Poloxamere; Rizinusöl- und Mineralölderivate; Sebazatester wie beispielsweise Dibutylsebazat), Triacetin, Fettsäureester wie beispielsweise Glycerolmonostearat oder Stearylalkohol oder Vitamin E TPGS (Tocopherolglycerylsuccinat) insbesondere Tocopherolglycerylsuccinat oder Polyethylenglykol 1500. Die Weichmacher können in Mengen von 0,1 bis 40 Gew.-%, bevorzugt 1 bis 20 Gew.-%, bezogen auf das Polymer, eingesetzt werden.

Die erzeugten festen Lösungen sind röntgenamorph. Der amorphe Zustand kann durch Röntgendiffraktion festgestellt werden. Der sogenannte "röntgenamorphe" Zustand der festen Lösungen bedeutet, dass der kristalline Anteil weniger als 5 Gew.-% beträgt.

Auch mit Hilfe eines DSC Thermogramms (Differential Scanning Calorimetry) kann der amorphe Zustand untersucht werden. Die erfindungsgemäßen festen Lösungen weisen keine Schmelzpeaks auf, sondern lediglich eine Glasübergangstemperatur, die bei den erfindungsgemäßen festen Lösungen auch von der Art des eingesetzten Wirkstoffs abhängt. Die Glasübergangstemperaturen, gemessen bei einer Heizrate von 20K/min, liegen überwiegend im Bereich von 50 bis 170 °C.

Bei der Herstellung der erfindungsgemäßen festen Dosierungsformen können gegebenenfalls übliche pharmazeutische Hilfsstoffe mitverarbeitet werden. Dabei handelt es sich um Stoffe aus der Klasse der Füllstoffe, Weichmacher, Löslichkeitsvermittler, Bindemittel, Silikate sowie Spreng- und Adsorptionsmittel, Schmiermittel, Fließmittel, Farbstoffe, Stabilisatoren wie Antioxidantien, Netzmittel, Konservierungsmittel, Formentrennmittel, Aromen oder Süßstoffe, bevorzugt um Füllstoffe, Weichmacher und Löslichkeitsvermittler.

Als Füllstoffe können z.B. anorganische Füllstoffe wie Oxide von Magnesium, Aluminium, Silicium, Titan- oder Calciumcarbonat, Calcium- oder Magnesiumphosphate oder organische Füllstoffe wie Lactose, Saccharose, Sorbit, Mannit zugesetzt werden.

Als Weichmacher eignen sich beispielsweise Triacetin, Triethylcitrat, Glycerolmonostearat, niedermolekulare Polyethylenglykole oder Poloxamere.

Als zusätzliche Löslichkeitvermittler eignen sich grenzflächenaktive Substanzen mit einem HLB-Wert (HydrophilicLipophilicBalance) größer 11, beispielsweise mit 40 Ethylenoxid-Einheiten ethoxiliertes hydriertes Ricinusöl (Cremophor® RH 40), mit 35 Ethylenoxid-Einheiten ethoxiliertes Ricinusöl (Cremophor eL), Polysorbat 80, Poloxamere oder Natriumlaurylsulfat.

Als Schmiermittel können Stearate von Aluminium, Calcium, Magnesium und Zinn, sowie Magnesiumsilikat, Silikone und ähnliche verwendet werden.

Als Fließmittel können beispielsweise Talk oder kolloidales Siliciumdioxid eingesetzt werden.

Als Bindemittel eignet sich zum Beispiel mikrokristalline Cellulose.

Als Sprengmittel können quervernetztes Polyvinylpyrrolidon, quervernetzte Natriumcarboxymethylcellulose oder quervernetzte Natriumcarboxymethylstärke eingesetzt werden. Als Stabilisatoren können alle Stoffe mit antioxidativen Eigenschaften fungieren wie z.B. Ascorbinsäure, Butylhydroxytoluol oder Tocopherol.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandiodid oder Talkum, um die Lichtdurchlässigkeit zu erhöhen und um Farbstoffe einzusparen.

Die erfindungsgemäßen Formulierungen lassen sich zu vielen verschiedenen Darreichungsformen formulieren, wie z. B. Tabletten, Kapseln, Granulate, Pulver, Drug Delivery Systeme, Lösungen, Suppositorien, transdermale Systeme, Cremes, Gele, Lotionen, Injektionslösungen, Tropfen, Säfte, Sirupe,

Durch den Zusatz von thermoplastischen Retardierungsmitteln wie Polyvinylacetat-Homopolymeren oder Formulierungen solcher Polyvinylacetat-Homopolymeren, weiterhin als Retardpolymeren auf Acrylatbasis bekannten Eudragit®-Typen RS, RL oder NE oder NM, lässt sich die Freisetzung verlangsamen. Auf diese Weise können verlässlich freisetzende Retardformen schwerlöslicher Arzneistoffe hergestellt werden.

Die erfindungsgemäßen Formulierungen besitzen eine ausgezeichnete Verarbeitbarkeit zu Darreichungsformen, insbesondere hinsichtlich der Tablettierbarkeit. Daher lassen sich Tabletten mit einem Durchmesser von 10mm und 300mg Gewicht herstellen, die eine Bruchfestigkeit von über 200N besitzen. Das wasserlösliche Polymere wirkt gleichzeitig als Bindemittel und verleiht der Tablettenrezeptur eine enorme Plastizität.

Aufgrund der höheren Löslichkeit ist die Bioverfügbarkeit ebenfalls höher und auch viel reproduzierbarer, d.h. es gibt geringere interindividuelle Schwankungen.

Die festen Lösungen lassen sich auch bei höheren Temperaturen durch Schmelzextrusion verarbeiten, ohne dass es zu einer Veränderung der Säurezahl kommt.

**Beispiele**

Abkürzungen/Methoden

Röntgendiffraktometrie

Messgerät: Diffraktometer D 8 Advance mit 9-fach Probenwechsler (Fa.Bruker/AXS)

Messart: $\theta$- $\theta$ Geometrie in Reflexion

Winkelbereich 2 Theta: 2-80°

Schrittweite: 0,02°

Messzeit pro Winkelschritt: 4,8s

Divergence Slit: Göbelspiegel mit 0,4 mm Steckblende

Antiscattering Slit: Sollerspalt

Detektor: Sol-X Detektor

Temperatur: Raumtemperatur

Generatoreinstellung: 40kV/50mA

Die Trübungsmessung erfolgte gemäß ISO 7027 durch Verhältnismessung von Streulicht und Transmission.

Die Glasübergangstemperaturen wurden bei einer Heizrate von 20K/min bestimmt, Einwaage. 8,5 mg.

Prozentangaben beziehen sich, soweit nicht anders angegeben, auf Gewichtsprozent.

Beispiel 1

Copolymer aus 80 Gew.-% N-Vinylpyrrolidon (VP) und 20 Gew.-% Acrylsäure (AS)

Apparatur:

2 l HWS Topf mit Ankerrührer, Rückflußkühler, Stickstoffeinleitung (über Flüssigkeits-spiegel) und temperierbarem Ölbad. 2 Zulaufgefäße je 1000 ml. Temperaturmessung im Polymerisationsgefäß und im Ölbad jeweils via Pt100 Fühler.

| Zuläufe | Menge | Einsatzstoff |
|---------|-------|--------------|
| Vorlage | 210,0 g | iso-Propanol |
| | 30,0 g | N-Vinylpyrrolidon |
| | 10,6 g | Teilmenge von Zulauf 2 |
| Zulauf 1 | 399,0 g | iso-Propanol |
| | 210,0 g | N-Vinylpyrrolidon |
| | 60,0 g | Acrylsäure |
| | | |
| Zulauf 2 | 100,0 g | iso-Propanol |
| | 6,0 g | tert.-Butylperpivalat, 75%ig |

Die Vorlage wurde unter leichtem Stickstoffstrom auf 75°C Innentemperatur aufgeheizt. Bei Erreichen der 75°C Innentemperatur wurde die Teilmenge des Zulaufes 2 zugegeben. Danach wurden der Zulauf 1 und die Restmenge des Zulaufs 2 gestartet. Der Zulauf 1 wurde in 6 Stunden und die Restmenge des Zulaufs 2 in 9 Stunden zudosiert.

Abschließend wurde das Reaktionsgemisch noch einer Wasserdampfdestilliation unterzogen, um das Lösungsmittel Isopropanol zu verdrängen.

| | |
|---|---|
| Feststoffgehalt FG (Gew.%) | 30,5 |
| K-Wert (5 %ig in Wasser) | 16,5 |
| FNU-Wert (5 %ig in Wasser) | 0,7 |
| Tg (°C) | 165 (gemessen mittels Differential Scanning Calorimetry DSC; berechnet 150 °C, s.o.) |
| Isopropanol (ppm) | 2800 |

Aussehen: Wasserklare niedrigviskose wässrige Lösung.

Beispiel 2

Es wurde wie in Beispiel 1 gearbeitet, allerdings wurde der Zulauf 1 in 4 Stunden und die Restmenge des Zulaufs 2 in 6 Stunden zudosiert.

Nach dem Ende des Zulaufes 2 wurde noch 1 Stunde bei einer Innentemperatur von 75°C nachpolymerisiert.

| | |
|---|---|
| FG (Gew.%) | 30,8 |
| K-Wert (5 %ig in Wasser) | 17,8 |
| FNU-Wert (5 %ig in Wasser) | 0,6 |
| Tg (°C) | 164 (DSC) |
| Isopropanol (ppm) | 2600 |

Aussehen: Wasserklare niedrigviskose wässrige Lösung.

Beispiel 3

Copolymer aus 70 Gew.-% VP und 30 Gew.% AS

Die Herstellung erfolgte analog Beispiel 1, wobei der Zulauf 1 180 g N-Vinylpyrrolidon und 90 g Acrylsäure enthielt.

| | |
|---|---|
| FG (Gew.%) | 30,4 |
| K-Wert *(5 %ig in Wasser)* | 15,8 |
| FNU-Wert (5 %ig in Wasser) | 1,5 |
| Tg (°C) | 144 (berechnet) |
| Isopropanol (ppm) | 2200 |

Aussehen: Wasserklare niedrigviskose wässrige Lösung.

Beispiel 4

Copolymer aus 90 Gew.-% VP und 10 Gew.% AS

Die Herstellung erfolgte analog Beispiel 1, wobei der Zulauf 1 240 g N-Vinylpyrrolidon und 30 g Acrylsäure enthielt.

| | |
|---|---|
| FG (Gew.%) | 30,3 |
| K-Wert *(5 %ig in Wasser)* | 16,7 |
| FNU-Wert (5 %ig in Wasser) | 0,6 |
| Tg (°C) | 156 (berechnet) |
| Isopropanol (ppm) | 2500 |

Aussehen: Wasserklare niedrigviskose wässrige Lösung.

Beispiel 5

Copolymer aus 85 Gew.-% VP und 15 Gew.% AS

Die Herstellung erfolgte analog Beispiel 1, wobei der Zulauf 1 225 g N-Vinylpyrrolidon und 45 g Acrylsäure enthielt.

| | |
|---|---|
| FG (Gew.%) | 30,5 |
| K-Wert (5 %ig in Wasser) | 16,4 |
| FNU-Wert (5 %ig in Wasser) | 0,7 |
| Tg (°C) | 153 (berechnet) |
| Isopropanol (ppm) | 2100 |

Aussehen: Wasserklare niedrigviskose wässrige Lösung.

**Vergleichsbespiel**

Copolymer aus 50 Gew.-% VP und 50 Gew.% AS

Die Herstellung erfolgte analog Beispiel 1, wobei der Zulauf 1 120 g Vinylpyrrolidon und 150 g Acrylsäure enthielt.

| | |
|---|---|
| FG (Gew.%) | 31,5 |
| K-Wert (5 %ig in Wasser) | 12,8 |
| FNU-Wert (5 %ig in Wasser) | 3,1 |
| Tg (°C) | 132 (berechnet) |

(fortgesetzt)

| | |
|---|---|
| Isopropanol (ppm) | 3000 |

Das so erhaltene Polymer mit einem FNU-Wert von 3,1 war bei visueller Begutachtung nicht mehr "wasserklar" sondern leicht trübe.

Beispiel 6

Herstellung einer festen Lösung durch Lösungsverfahren

1250 g des Copolymers VP/AS (80/20) wurden zusammen mit 416,7 g Haloperidol (Base) in 5999,3 g eines Lösemittelgemischs bestehend aus 50 % (2999,7 g) Ethanol und 50 % (2999,7 g) Isopropanol unter Rühren bei Raumtemperatur gelöst. Die organische Lösung hatte einen Gesamtfeststoffgehalt von 21,7 %. Die Lösung wurde anschließend unter den folgenden Bedingungen in einem Laborsprühtrockner sprühgetrocknet:

Trocknungsgas: Stickstoff; 30 Nm3/h

Einlasstemperatur (inlet temperature): 155°C

Auslasstemperatur (outlet temperature): 75°C

Zerstäubungsdüse: 1,4 mm Zweistoffdüse

Zerstäubungsgas / Zerstäubungsdruck: Stickstoff / 2 bar abs.

Fördergeschwindigkeit (liquid flow rate): 977,9 g/h

Produktabscheidung: Zyklon

Die folgende Tabelle gibt eine Übersicht über die Eigenschaften des sprühgetrockneten Polymeren Salzes Haloperidol-VP/AS nach der Sprühtrocknung aus organischer Lösung (Ethanol : Isopropanol 1:1).

| | |
|---|---|
| Restfeuchte (gemessen bei 105°C) | 1,75 % |
| Arzneistoffgehalt (gemessen, UV/VIS bei 248 nm) | 24,5 % |
| Zustand Arzneistoff (XRD) | röntgenamorph |
| Glasübergangstemperatur | 129°C (kein Schmelzpeak) |

Weder im DSC Thermogramm noch in der Röntgendiffraktometrie waren kristalline Wirkstoffanteile zu erkennen. Es kann somit davon ausgegangen werden, dass es sich um ein amorphes Pulver handelt. Die Freisetzung des Wirkstoffes aus dem sprühgetrockneten polymeren Salz wurde in VE-Wasser durchgeführt. Die Einwaage wurde auf 100 mg Haloperidol pro 250 ml Freisetzungsmedium berechnet. Das entsprechend abgewogene polymere Salz bzw. die kristalline Substanz wurden in Hartgelatinekaspeln abgefüllt. Die folgende Tabelle zeigt die Ergebnisse der Freisetzung im Vergleich zum kristallinen Arzneistoff.

| Freisetzung Polymeres Salz Haloperidol-VP/AS (hergestellt aus organischer Lösung) in VE-Wasser | 0 min | 0 % |
|---|---|---|
| | 2 min | 1,4 % |
| | 4 min | 62,5 % |
| | 6 min | 84,8 % |
| | 8 min | 89,0 % |
| | 10 min | 95,4 % |
| | 30 min | 98,3 % |
| | 60 min | 98,6 % |
| | 120 min | 98,6 % |
| Kristallines Haloperidol in VE-Wasser | 0 min | 0,0 % |
| | 2 min | 0,1 % |
| | 4 min | 0,2 % |
| | 6 min | 0,3 % |
| | 8 min | 0,4 % |
| | 10 min | 0,6 % |
| | 30 min | 1,5 % |
| | 60 min | 1,8 % |
| | 120 min | 2,3 % |

Herstellung der festen Lösung durch Schmelzeverfahren: Die Verarbeitung eines Polymers gemäß Beispiel 1 mit verschiedenen Wirkstoffen erfolgte mit einem Doppelschneckenextruder: Pharma Extruder, Haake PolyLab OS, RheoDrive 7 (Messantrieb); Haake Rheomex OS, PTW 16 mm (Messvorsatz); Gravimetrische Dosiereinheit Brabender Doppeldosierschnecke Typ DDSR 20, Software AIO-Control

Schneckentyp L/D 40

Düsentyp: 3 mm, Lochdüse
Drehzahl: 200/min
Drehmoment: 50 Nm
Dosierung: 1 kg/h
Kühlband für Extruder Typ 557-2680 (Thermo Fischer)
Granulator Thermo Electron Typ 554-1345
TubeMill (Modell Tube Mill control, IKA); Vermahlung der Extrudatstücke bei 25000 U/min

Die jeweilige Extrusionstemperatur ist in der nachstehenden Tabelle aufgeführt.

| Probenbezeichnung | Formulierung | | Extrusionstemperatur | | | | |
|---|---|---|---|---|---|---|---|
| | | | Zone 1 | Zone 2 | Zone 3 | Zone 4-9 | Zone 10 |
| Probe 1 | VP/AS (80/20) | 100 % | 50°C | 80°C | 195°C | 270°C | 250°C |
| Probe 2 | VP/AS (80/20) | 100 % | 50°C | 80°C | 170°C | 300°C | 230°C |
| Probe 12 | VP/AS (80/20) Kolliphor TPGS | 90 % 10 % | 50°C | 80°C | 170°C | 225°C | 190°C |
| Probe 13 | VP/AS (80/20) | 90 % | 50 °C | 80 °C | 170°C | 225°C | 190°C |

(fortgesetzt)

| Probenbezeichnung | Formulierung | | Extrusionstemperatur | | | | |
|---|---|---|---|---|---|---|---|
| | | | Zone 1 | Zone 2 | Zone 3 | Zone 4-9 | Zone 10 |
| | PEG 1500 | 10 % | | | | | |
| Probe 14 | VP/AS (80/20)<br>Kolliphor TPGS<br>Loperamid (Base) | 85 %<br>10 %<br>5 % | 50 °C | 80 °C | 170°C | 225°C | 190°C |
| Probe 15 | VP/AS (80/20)<br>Kolliphor TPGS<br>Loperamid (Base) | 80 %<br>10 %<br>10 % | 50 °C | 80 °C | 170°C | 225°C | 190°C |
| Probe 16 | VP/AS (80/20)<br>PEG 1500<br>Loperamid Base | 85 %<br>10 %<br>5 % | 50 °C | 80 °C | 170°C | 225°C | 190°C |
| Probe 17 | VP/AS (80/20)<br>PEG 1500<br>Loperamid Base | 80 %<br>10 %<br>10 % | 50 °C | 80 °C | 170°C | 225°C | 190°C |

Solubilisierung

Die Wirkstofffreisetzung aus den Proben 14, 15, 16 und 17 wurden im Freisetzungsgerät mit automatischer spektroskopischer der Messung (00501 FEXXX-D) geprüft. Die Extrudate mit 5% Wirkstoffgehalt wurden zu Beginn des Versuches direkt in das Freisetzungsmedium gegeben. Die Extrudate mit 10% Wirkstoffgehalt wurden mit 15 % (m/m) Avicel PH 101 (mikrokristalline Cellulose) und von 15 % (m/m) Kollidon CL (Sprengmittel) gemischt. Die Abmischungen wurden in Hartgelatine-Kapsel gefüllt und aus diesen freigesetzt.

Durchführung einer Freisetzung (2 Stunden):

Bedingungen 0 - 2 Stunden: Medium:

• 250 ml VE-Wasser;

• Paddle Apparatur 50 U/min;

• Temperatur 37°C

• Gehaltsbestimmung UV/VIS mit Küvette 1 cm Dicke

• Wirkstoffmenge 250 mg

Die Ergebnisse der Freisetzungen sind in den folgenden Tabellen und Diagrammen dargestellt. Die Freisetzung ist in allen Fällen vollständig. Das kristalline Loperamid (Base) zeigte keinerlei Auflösung im Freisetzungsmedium bzw. es konnte keine aufgelöste Menge Loperamid mit der verwendeten spektroskopischen Methode detektiert werden.

| Zeit [min] | Freigesetztes Loperamid [%] | | | |
|---|---|---|---|---|
| | Probe 14 5% Loperamid TPGS | Probe 15 1 0% Loperamid TPGS | Probe 16 5% Loperamid PEG 1500 | Probe 16 10% Loperamid PEG 1500 |
| 0 | 0,0 % | 0,0 % | 0,0 % | 0,0 % |
| 2 | 57,2 % | 5,7 % | 58,2 % | 0,3 % |
| 4 | 86,4 % | 20,0 % | 75,7 % | 27,6 % |

(fortgesetzt)

| Zeit [min] | Freigesetztes Loperamid [%] | | | |
|---|---|---|---|---|
| | Probe 14 5% Loperamid TPGS | Probe 15 1 0% Loperamid TPGS | Probe 16 5% Loperamid PEG 1500 | Probe 16 10% Loperamid PEG 1500 |
| 6 | 92,8 % | 48,7 % | 81,5 % | 33,1 % |
| 8 | 95,4 % | 69,6 % | 84,5 % | 47,2 % |
| 10 | 97,0 % | 76,6 % | 87,0 % | 58,5 % |
| 30 | 101,6 % | 90,8 % | 96,7 % | 89,0 % |
| 60 | 100,3 % | 95,0 % | 99,3 % | 93,4 % |
| 120 | 97,6 % | 98,0 % | 101,9 % | 96,1 % |

Tablettenherstellung

Das in Beispiel 6 durch Sprühtrocknung hergestellte polymere Salz mit einem Haloperidolgehalt von 24,5% (m/m) wurde zur Herstellung von Tabletten verwendet.

Rezeptur:

| Ludipress | 72,1 % | 360,5 mg |
|---|---|---|
| Polymeres Salz (gemäß Beispiel 6, Haloperidolgehalt 24,5%) | 20,4 % | 102,0 mg |
| Aerosil 200 | 2,0 % | 10,0 mg |
| Magnesiumstearat | 0,5 % | 2,5 mg |
| Kollidon CL | 5,0 % | 25,0 mg |
| | 100,0 % | 500,0 mg |

Die Tablettierung erfolgte auf einer Korsch XP1 Exzenterpresse, Stempelform 11 mm gewölbt bei einer Presskraft: 10kN. Die Freisetzung des Wirkstoffes aus den hergestellten Tabletten wurde in VE-Wasser durchgeführt. Die Einwaage wurde auf 100 mg Haloperidol pro 250 ml Freisetzungsmedium berechnet, entsprechend 4 Tabletten pro 250 ml Freisetzungsmedium. Die folgende Tabelle zeigt die Ergebnisse der Freisetzung im Vergleich zum kristallinen Arzneistoff.

| Freisetzung Polymeres Salz Haloperidol-VP/AS (sprühgetrocknet aus organischer Lösung, formuliert und verpresst in Tabletten) in VE-Wasser | 0 min | 0 % |
|---|---|---|
| | 2 min | 5,4 % |
| | 4 min | 12,9 % |
| | 6 min | 20,7 % |
| | 8 min | 27,8 % |
| | 10 min | 34,5 % |
| | 30 min | 82,2 % |
| | 60 min | 97,4 % |
| | 120 min | 97,9 % |

Bestimmung der Bindungsart in einer festen Lösung

Hierbei wurde eine erfindungsgemäße feste Lösung aus Loperamid und dem Copolymer gemäß Beispiel 1, hergestellt wie in Beispiel 6 beschrieben, mit einem festen Salz aus Loperamid und dem gleichen Copolymer sowie mit einer festen Lösung aus Loperamid und PVP K17 verglichen. Die feste Lösung aus Loperamid und Copolymer wurde durch Eindampfen einer Lösung der Substanzen in THF/Methanol erhalten. Die feste Lösung von Loperamid und PVP wurde wie folgt erhalten:1 Teil Loperamid (Base) und 9 Teile PVP K17 wurden in DMF gelöst. Die Feststoffkonzentration betrug 25 % (m/m). Die Lösung wurde in eine Kautschukplatte ausgegossen und für 48 Stunden im Vakuumtrockenschrank bei 50°C und 10 mbar getrocknet. Die erhaltene feste Lösung wurde anschließend aus der Kautschukplatte entfernt und in einer TubeMill (Modell Tube Mill control, IKA) bei 25000 U/min zu einem Pulver vermahlen.

Reaktionskalorimetrie

Für die Untersuchungen wurde ein Nano ITC (Isothermal Titration Calorimeter) der Firma TA Instruments verwendet. In der Messzelle wurde jeweilige Polymer vorgelegt. Dazu wurde Loperamid sukzessive zugegeben. Sobald eine Reaktion stattfindet, verändert sich die Temperatur der Probe. Dieser Temperaturunterschied wurde registriert und über ein Peltierelement ausgeglichen. Die zum Ausgleich benötigte elektrische Energie wurde erfasst. Diese Energiemenge ist vom Betrag identisch mit der entstandenen oder verbrauchten Wärmemenge der Reaktion. Eingebettet sind die beiden Zellen, Messzelle und Referenzzelle in ein hochstabiles Temperierbad (+-0,0002 K bei 25°C). Während des Versuchs wurde gerührt.

In Folge der geringen Wärmemengen, die hier gemessen werden, ist es notwendig Referenzmessungen durchzuführen. In diesen Falle die Verdünnungsreaktion der Polymere. Die bei der Verdünnungsreaktion gemessene Wärmemenge wurde von der Originalmessung subtrahiert. Der erhaltende Wert ist die Reaktionsenthalpie der Reaktion Wirkstoff- Polymer.

Zur Bestimmung der Reaktionsenthalpie wurden die Polymere jeweils in einer Konzentration von 3g/L angesetzt und jeweils 1ml vorgelegt. Die Loperamid-Base wurde in einer Konzentration von 25mg/l angesetzt und in 5µl Schritten zugegeben. Nach jeder Zugabe wurde 600s gewartet bis die Reaktion beendet war. Gemessen wurde bei 25°C. Als Lösungsmittel diente eine Wasser/Ethanol - Mischung (9:1).

Die Polymere wurden in großem Überschuss vorgelegt, sodass sichergestellt war, dass jedes zugegebene Molekül einen entsprechenden Reaktionspartner finden konnte.

Lösungskalorimetrie

Durchgeführt wurde diese Untersuchung mit einem TAM III mit SolCal-Einsatz der Fa. TA-Instruments. Dazu wurde das Lösungsmittel vorgelegt. Die zu lösende Probe wurde in einer Glasampulle hermetisch eingeschlossen. Die Ampulle wurde in das Lösungsmittel eingebracht und Temperaturgleichheit hergestellt. Die Lösungskalorimetrie wurde bei 25°C (Nenntemperatur) durchgeführt.

In einem nächsten Schritt wurde die Arbeitstemperatur auf eine Temperatur im Bereich von 0,3 K unter die Nenntemperatur gebracht. Anschließend wurde das Angleichen der Arbeitstemperatur an die Nenntemperatur gemessen der Kurvenverlauf bestimmt und durch gezielte Energiezufuhr (elektrische Energie = thermische Energie) kalibriert. Die Formulierungen wurden in eine Bruchampulle eingewogen (20-140mg) und in 100 mL eines Gemischs aus Wasser/ Ethanol im Volumenverhältnis 9:1 eingebracht.

Dann wurde die Ampulle auf einem Dorn in der Messzelle gebrochen. Die zu messende Substanz wurde dadurch im Lösungsmittel freigesetzt. Nach Abzug der Basislinie des Temperaturverlaufes und der Umrechnung der Temperatur in die entsprechende Wärmemenge erhielt man eine Heat- Flow- Kurve, Wärmemenge vs. Zeit. Nach Integration der Kurve und Bezugnahme auf die Molekulargewichte, wurde die Lösungsenthalpie der zu untersuchenden Substanz erhalten.

Die Lösungswärme aller Proben wurde doppelbestimmt. Von der Lösungswärme der Formulierung wurde anteilig die Enthalpie des Polymers, die in einer separaten Messung bestimmt wurde, subtrahiert.

Die nachstehende Tabelle zeigt die Ergebnisse der Messungen zur Lösungsenthalpie der einzelnen Loperamid-Polymer- Formulierungen. Spalte A zeigt ist die Gesamtwärmemenge $Q_{Ges}$ für die jeweilige Formulierung. Spalte B weist die anteilige Wärmemenge des Polymeren $Q_{Poly}$ aus. Die Differenz ergibt die Wärmemenge $Q_{Wirkstoff}$ des Wirkstoffes auf die Gesamteinwaage bezogen (Spalte C) bzw. auf den Wirkstoffanteil bezogen (Spalte D). Der Wert in der Spalte E beziffert die Lösungsenthalpie bzw. Bindungsenthalpie (Delta H) des Loperamids.

| | A | B | C | D | E |
|---|---|---|---|---|---|
| | $Q_{ges}$ J/g* | $Q_{Poly}$ J/g* | $Q_{Wirk- stoff}$ J/g * | $Q_{wirk- stoff}$ J/g ** | Delta H kJ/mol |
| 10,4% Loperamid Base VP/AS erhalten aus VE-Wasser | 263 | 202 | 61 | 586 | -279 |
| 32,92% Loperamid Base VP/AS erhalten aus THF/ Methanol | 154 | 151 | 3 | 8 | -4 |

(fortgesetzt)

|  | A | B | C | D | E |
|---|---|---|---|---|---|
| feste Lösung 10% Loperamid/PVP K17 | 241 | 235 | 6 | 57 | -27 |
| Polymer VP/AS 80/20 | 226 |  |  |  |  |
| Kollidon® 17 (PVP K17) | 261 |  |  |  |  |
| * Auf Gesamteinwaage bezogen<br>**Auf Wirkstoffanteil bezogen |  |  |  |  |  |

Bestimmung der Beladbarkeit der festen Lösungen

**Patentansprüche**

1. Verwendung eines wasserlöslichen Polymers, welches im pH-Bereich von 1 bis 13 eine Wasserlöslichkeit größer von 5 % (m/m) aufweist und durch radikalisch initiierte Polymerisation einer Monomerenmischung aus i) 70 bis 90 Gew.-% N-Vinylpyrrolidon und ii) 10 bis 30 Gew.-% Acrylsäure, wobei die Summe von i) und ii) 100 Gew.-% entspricht, erhalten wird, zur Formulierung von in Wasser schwerlöslichen basischen Wirkstoffen, wobei die Wirkstoffe in nicht geladener Form oder als Hydrochlorid eine Löslichkeit von kleiner 0.1 % (m/m) in Wasser, künstlichem Darmsaft oder Magensaft aufweisen.

2. Verwendung nach Anspruch 1, zur Solubilisierung des basischen Wirkstoffs.

3. Verwendung nach einem der Ansprüche 1oder 2, wobei der Wirkstoff mindestens eine und höchstens zwei zur Salzbildung befähigten Gruppen aufweist.

4. Verwendung nach einem der Ansprüche 1 bis 3, wobei das wasserlösliche Salz eine höhere Wasserlöslichkeit als der Wirkstoff und das entsprechende Hydrochlorid des Wirkstoffes aufweist.

5. Verwendung nach einem der Ansprüche 1 bis 4, wobei das wasserlösliche Polymer welches im pH-Bereich von 1 bis 13 eine Wasserlöslichkeit größer 10 % (m/m) aufweist.

6. Verwendung nach einem der Ansprüche 1 bis 5, wobei das wasserlösliche Polymer in 5 gew.-%iger wässriger Lösung einen K-Wert nach Fikentscher von kleiner 30 aufweist.

7. Verwendung nach einem der Ansprüche 1 bis 6, wobei das wasserlösliche Polymer in 5 gew.-%iger wässriger Lösung einen K-Wert nach Fikentscher von kleiner 20 aufweist.

8. Verwendung nach einem der Ansprüche 1 bis 7, enthaltend ein wasserlösliches Polymer, welches durch radikalisch initiierte Polymerisation einer Monomerenmischung aus i) 79 bis 81 Gew.-% N-Vinylpyrrolidon und ii) 18 bis 22 Gew.-% Acrylsäure, wobei die Summe von i) und ii) 100 Gew.-% entspricht, erhalten wird.

9. Verwendung nach einem der Ansprüche 1 bis 8, enthaltend eine wasserlösliches Polymer, welches durch radikalisch initiierte Polymerisation einer Monomerenmischung aus i) 80 Gew.-% N-Vinylpyrrolidon und ii) 20 Gew.-% Acrylsäure, wobei die Summe von i) und ii) 100 Gew.-% entspricht erhalten wird.

10. Verwendung nach einem der Ansprüche 1 bis 9, wobei die Formulierungen in Form fester Lösungen vorliegen.

11. Verwendung nach einem der Ansprüche 1 bis 10, wobei das wasserlösliche Polymer als Bindemittel wirkt.

12. Dosierungsformen, enthaltend wasserlösliche Formulierungen von in Wasser schwerlöslichen Wirkstoffen und einem Polymer gemäß einem der Ansprüche 1 bis 11, wobei die Formulierungen aus einem basischen Wirkstoff, welcher in nicht geladener Form oder als Hydrochlorid eine Löslichkeit von kleiner 0.1 % (m/m) in Wasser, künstlichem Darmsaft oder Magensaft aufweist, und einem wasserlöslichen Polymer, welches im pH-Bereich von 1 bis 13 eine Wasserlöslichkeit größer von 10 % (m/m) aufweist, und durch radikalisch initiierte Polymerisation einer Monomerenmischung aus i) 78 bis 82 Gew.-% N-Vinylpyrrolidon und ii) 18 bis 22 Gew.-% Acrylsäure, wobei die Summe von i) und ii) 100 Gew.-% entspricht, erhalten wird, bestehen.

13. Dosierungsformen nach Anspruch 12, enthaltend zusätzlich pharmazeutische Hilfsstoffe.

14. Dosierungsformen nach Anspruch 13 oder 14, hergestellt durch Verpressung.

**Claims**

1. The use of a water-soluble polymer having a solubility in water of greater than 5% (m/m) in the pH range of 1 to 13 and which is obtained by free-radically initiated polymerization of a monomer mixture of i) 70 to 90% by weight N-vinylpyrrolidone and ii) 10 to 30% by weight acrylic acid, wherein the sum total of i) and ii) corresponds to 100% by weight, for the formulation of basic active ingredients sparingly soluble in water, wherein the active ingredients in uncharged form or as hydrochloride have a solubility of less than 0.1% (m/m) in water, artificial intestinal juice or gastric juice.

2. The use according to claim 1 for solubilizing the basic active ingredient.

3. The use according to either of claims 1 and 2, wherein the active ingredient has at least one and at most two groups capable of salt formation.

4. The use according to any of claims 1 to 3, wherein the water-soluble salt has a higher water solubility than the active ingredient and the corresponding hydrochloride of the active ingredient.

5. The use according to any of claims 1 to 4, wherein the water-soluble polymer has a solubility in water of greater than 10% (m/m) in the pH range of 1 to 13.

6. The use according to any of claims 1 to 5, wherein the water-soluble polymer in 5% by weight aqueous solution has a Fikentscher K-value of less than 30.

7. The use according to any of claims 1 to 6, wherein the water-soluble polymer in 5% by weight aqueous solution has a Fikentscher K-value of less than 20.

8. The use according to any of claims 1 to 7, comprising a water-soluble polymer which is obtained by free-radically initiated polymerization of a monomer mixture of i) 79 to 81% by weight N-vinylpyrrolidone and ii) 18 to 22% by weight acrylic acid, wherein the sum total of i) and ii) corresponds to 100% by weight.

9. The use according to any of claims 1 to 8, comprising a water-soluble polymer which is obtained by free-radically initiated polymerization of a monomer mixture of i) 80% by weight N-vinylpyrrolidone and ii) 20% by weight acrylic acid, wherein the sum total of i) and ii) corresponds to 100% by weight.

10. The use according to any of claims 1 to 9, wherein the formulations are in the form of solid solutions.

11. The use according to any of claims 1 to 10, wherein the water-soluble polymer acts as binder.

12. A dosage form comprising water-soluble formulations of active ingredients sparingly soluble in water and a polymer according to any of claims 1 to 11, wherein the formulations consist of a basic active ingredient, which in uncharged form or as the hydrochloride has a solubility of less than 0.1% (m/m) in water, artificial intestinal juice or gastric juice, and a water-soluble polymer having a solubility in water of greater than 10% (m/m) in the pH range of 1 to 13 and which is obtained by free-radically initiated polymerization of a monomer mixture of i) 78 to 82% by weight N-vinylpyrrolidone and ii) 18 to 22% by weight acrylic acid, wherein the sum total of i) and ii) corresponds to 100% by weight.

13. The dosage form according to claim 12, additionally comprising pharmaceutical auxiliaries.

14. The dosage form according to claim 13 or 14, prepared by compression.

**Revendications**

1. Utilisation d'un polymère soluble dans l'eau, qui présente dans la plage de pH allant de 1 à 13 une solubilité dans l'eau supérieure à 5 % (m/m) et qui est obtenu par polymérisation initiée radicalairement d'un mélange de monomères constitué par i) 70 à 90 % en poids de N-vinylpyrrolidone et ii) 10 à 30 % en poids d'acide acrylique, la somme de i) et ii) correspondant à 100 % en poids, pour la formulation d'agents actifs basiques difficilement solubles dans l'eau, les agents actifs présentant sous forme non chargée ou sous la forme d'un chlorhydrate une solubilité inférieure à 0,1 % (m/m) dans l'eau, le suc intestinal ou le suc gastrique artificiel.

2. Utilisation selon la revendication 1, pour la solubilisation de l'agent actif basique.

3. Utilisation selon l'une quelconque des revendications 1 ou 2, dans laquelle l'agent actif comprend au moins un et au plus deux groupes aptes à la formation de sels.

4. Utilisation selon l'une quelconque des revendications 1 à 3, dans laquelle le sel soluble dans l'eau présente une solubilité dans l'eau plus élevée que l'agent actif et le chlorhydrate correspondant de l'agent actif.

5. Utilisation selon l'une quelconque des revendications 1 à 4, dans laquelle le polymère soluble dans l'eau présente dans la plage de pH allant de 1 à 13 une solubilité dans l'eau supérieure à 10 % (m/m).

6. Utilisation selon l'une quelconque des revendications 1 à 5, dans laquelle le polymère soluble dans l'eau présente dans une solution aqueuse à 5 % en poids une valeur K selon Fikentscher inférieure à 30.

7. Utilisation selon l'une quelconque des revendications 1 à 6, dans laquelle le polymère soluble dans l'eau présente dans une solution aqueuse à 5 % en poids une valeur K selon Fikentscher inférieure à 20.

8. Utilisation selon l'une quelconque des revendications 1 à 7, contenant un polymère soluble dans l'eau, qui est obtenu par polymérisation initiée radicalairement d'un mélange de monomères constitué par i) 79 à 81 % en poids de N-vinylpyrrolidone, et ii) 18 à 22 % en poids d'acide acrylique, la somme de i) et ii) correspondant à 100 % en poids.

9. Utilisation selon l'une quelconque des revendications 1 à 8, contenant un polymère soluble dans l'eau, qui est obtenu par polymérisation initiée radicalairement d'un mélange de monomères constitué par i) 80 % en poids de N-vinylpyrrolidone, et ii) 20 % en poids d'acide acrylique, la somme de i) et ii) correspondant à 100 % en poids.

10. Utilisation selon l'une quelconque des revendications 1 à 9, dans laquelle les formulations se présentent sous la forme de solutions solides.

11. Utilisation selon l'une quelconque des revendications 1 à 10, dans laquelle le polymère soluble dans l'eau agit en tant que liant.

12. Formes posologiques, contenant des formulations solubles dans l'eau d'agents actifs difficilement solubles dans l'eau et d'un polymère selon l'une quelconque des revendications 1 à 11, les formulations étant constituées par un agent actif basique, qui présente sous forme non chargée ou sous la forme d'un chlorhydrate une solubilité inférieure à 0,1 % (m/m) dans l'eau, le suc intestinal ou le suc gastrique artificiel, et un polymère soluble dans l'eau, qui présente dans la plage de pH allant de 1 à 13 une solubilité dans l'eau supérieure à 10 % (m/m), et est obtenu par polymérisation initiée radicalairement d'un mélange de monomères constitué par i) 78 à 82 % en poids de N-vinylpyrrolidone, et ii) 18 à 22 % en poids d'acide acrylique, la somme de i) et ii) correspondant à 100 % en poids.

13. Formes posologiques selon la revendication 12, contenant en outre des adjuvants pharmaceutiques.

14. Formes posologiques selon la revendication 13 ou 14, fabriquées par compression.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0211268 A
- US 4997643 A
- US 4248855 A
- US 5736127 A
- US 4205060 A
- EP 0721785 A
- US 4853439 A
- WO 2007017452 A
- WO 2009074609 A

**In der Beschreibung aufgeführte Nicht-Patentliteratur**

- **H. UELZMANN.** *Journal of Polymer Science,* 1958, vol. XXXIII, 377-379
- **JANSSENS S. ; VAN DEN MOOTER G.** Review: Physical chemistry of solid dispersions. *Journal of Pharmacy and Pharmacology,* 2009, vol. 61, 1571-1586
- **VASCONCELOS et al.** Solid dispersions as strategy to improve oral bioavailability of poor water soluble drugs. *Drug Discovery Today,* 2006, vol. 12 (23/24), 1068-1075
- **AMIDON et al.** *Pharm. Res.,* vol. 12, 413-420